# EUROPEAN PATENT APPLICATION

(11) **EP 4 480 579 A1**
(43) Date of publication of application: **25.12.2024**
(21) Application number: 23180154.9
(22) Date of filing: 19.06.2023
(51) Int. Cl.: B01L 3/00, B01L 9/00, G01N 35/00

(54) **DEVICE FOR TESTING MULTIPLE ANALYTES IN LIQUID SAMPLE THROUGH LATERAL FLOW**

(71) Applicant: Zhejiang Orient Gene Biotech Co., Ltd, Anji Huzhou Zhejiang 313300 (CN)
(72) Inventor: LEI, Siyu, Huzhou (CN); FANG, Jianqiu, Huzhou (CN); ZHANG, Hua, Huzhou (CN)
(74) Representative: Piotrowicz, Pawel Jan Andrzej

(57) **Abstract**

The invention provides a device for testing an analyte in a fluid sample, and the device includes: a housing combined with an upper card and a lower card, where a supporting structure for supporting a lateral flow testing element is arranged on the lower card, the lateral flow testing element is configured to test the analyte in the fluid sample and includes an application pad for receiving a liquid sample, the supporting structure includes a supporting structure for supporting an entire sample application pad, and part of the supporting structure for supporting the sample application pad is inclined.

## Description

### BACKGROUND OF THE INVENTION

### Field of the Invention

The invention relates to a device for collecting and testing a liquid sample, in particular, to a device for collecting and testing an analyte in a liquid sample in the field of rapid diagnosis, such as a urine and saliva collection and testing device, and specifically to a device for testing multiple analytes in a same liquid sample through lateral flow.

### Description of the Related Art

The following description is merely an introduction of some background general knowledge and does not constitute any limitation to the invention.

In the field of in vitro diagnosis (IVD), chromatographic techniques are often used to diagnose and detect diseases and other items. For example, immune colloidal gold test strip, dry chemical test strip, immunofluorescence test strip, and the like all react with reagents after samples are pretreated based on the principle of chromatography, so as to finally obtain diagnosis results showing whether patients suffer from diseases. The function process of the immunofluorescence test strip is as follows: after samples (whole blood, plasma, and the like) are dripped into a sample application pad, liquid flows to an absorbent filter paper; the samples are treated in the sample application pad to filtrate erythrocytes and remove interfering substances and the like; when flowing through a conjugate pad, the samples immunobind with antigens and antibodies and carry fluorophores; when flowing through a nitrocellulose membrane, the samples specifically binds with antigens and antibodies bound thereon in advance; and fluorophores gathered on a testing line and a control line can reflect test results, and other interfering substances unbound are absorbed by the absorbent filter paper. Fluorescence immunochromatography has been widely used in the field of POCT detection in recent years because of its simple operation, strong specificity, high sensitivity, and quantification. However, in recent decades, most of immunochromatographic test cards can be used for detecting a single item only in a form of a single card with a single test strip. However, with the development of medical technologies, multiple targets need to be detected at the same time during diagnosis of diseases for more accurate determination, such as myocardial 3-item joint examination and myocardial 5-item joint examination. Under some circumstances, it is necessary to detect the status of multiple organs at the same time to determine the diseases, such as cardiopulmonary 5-item joint examination.

At present, the test device for detecting the presence or absence of an analyte in sample is widely used in hospitals or homes, and such a test device for rapid diagnosis includes one or more test strips, such as early pregnancy detection and drug abuse detection. Such test device for rapid diagnosis is very convenient, and can obtain a test result from the test strips after one minute or no at most about ten minutes. Drug detection is widely used by the drug control department, the Public Security Bureau, drug rehabilitation centers, physical examination centers, physical examination offices of national conscription, etc. The drug detection is diverse and frequent. Some detections are required to collect samples and then samples are detected in professional testing agencies or testing laboratories, and some detections need to be completed in the site in time, for example, roadsides, for example, persons who drive after drug use need to be tested on the spot (referred to as "Drug Driving"), to obtain the test results in time.

A lateral flow measuring device may include a housing having a sample port and a result window downstream of the sample port, and optionally a control window downstream of the result window. The sample port is suitable for receiving a specific amount of liquid buffer or a sample applied thereto, and the liquid buffer or the sample extends across a lateral flow path from the sample port to a downstream position via a lateral flow test strip in the housing. The housing may be made of any suitable materials such as molded plastics, and preferably materials having enough rigidity to provide support and stability for one or more lateral flow paths accommodated therein. An adhesive may be assembled onto the surface of the housing and faces a lateral flow matrix to help such matrix to be kept at an appropriate position in the housing.

WO2007/063423 discloses a lateral flow device, where the housing further includes one or more pressure bars, supports, and/or positioning pins for arranging a plurality of layers and strips in the housing and keeping the layers and strips at appropriate positions of an assembling device. For example, the top of the housing may be provided with a pressure bar for keeping an upstream part of a lower aspirating element at an appropriate position of a buffer hole and a pressure bar for keeping a downstream tail end of the lower aspirating element mutually contact with an upstream tail end of a main strip and keeping them at appropriate positions of an assembling device. In an embodiment, for example, when the top of the housing is made of molded plastics, these pressure bars and the top of the housing may be integrally formed. Alternatively, one or more pressure bars may be provided as separate elements.

WO2020/143219 further describes a device for testing multiple analytes by applying a sample, but the design of the device is more complex. A multi-step mechanism is provided in a sample application area and used to receive a liquid sample or a buffer. In addition, each step is designed with different heights. In fact, it is difficult to keep each product consistent in specific production. The main reason may be that different heights of step-shaped structures made of a water absorbent material cannot meet consistency for each batch. Although multiple items or analytes can be detected at the same time, the structural designs thereof are complex.

WO2008/110044 further discloses a device for testing multiple analytes by applying a sample once. However, a sample application hole and a test strip are arranged in a same width, an amount of applied samples needs to be relatively large and it is difficult to allow each test strip to contact with the sample at the same time, resulting in inaccurate test results and large time difference of test results obtained. Although that test strips are radially distributed for a small sample application hole is further disclosed, a sample can be applied once for detection on multiple different test strips, which still affects the detection accuracy.

The above lateral flow devices have the following defects: (1) the structural design is complex and the manufacturing cost is high; (2) most of them are operated by persons professionally trained; and if they are operated by persons not professionally trained, this leads to inaccurate detection; (3) although they are operated by persons professionally trained, this still leads to the problem of inaccurate test results. In view of the above technical problems in some conventional products, it is necessary to improve them and provide an alternative approach to solve the drawbacks of the prior art.

### BRIEF SUMMARY OF THE INVENTION

In order to overcome the defects in the prior art, the object of the invention is to provide a device for testing an analyte in a fluid sample. In particular, it is desired to provide a device for testing multiple analytes by applying a liquid sample once and a device capable of evenly distributing samples on two test strips by applying the samples once. A sample is applied once through a lateral immune detection device, so that the sample on each lateral flow test strip is substantially evenly distributed. In addition, even if an excessive amount of sample is applied, test results are not affected. This is particularly suitable for home self-testing.

In order to solve the problem, the invention provides a test device, and the device includes an upper card and a lower card, where a supporting structure for supporting a lateral flow testing element is arranged on the lower card, the supporting structure is provided with a plurality of testing elements, and each of the testing elements is capable of testing different analytes in a sample.

In some embodiments, the supporting structure for supporting a lateral flow testing element has a testing area for supporting the testing element, a label area, and a sample application area. In some embodiments, a supporting structure for supporting the sample application area is provided with a first supporting area and a second supporting area, where the second supporting area is obliquely arranged. In some embodiments, the first supporting area and a supporting structure for supporting the label area are located at a same horizontal position. The second supporting area is lower than the first supporting area and is obliquely arranged in a gradual decrease way.

In some embodiments, the lower card is provided with a plurality of supporting structures for supporting test strips, and the supporting structures all have a testing area for supporting a testing element, a label area, and a sample application area. Each of the supporting structures is provided with the structure for supporting the sample application area, and such structure is provided with a first supporting area and a second supporting area, where the second supporting area is obliquely arranged. In some embodiments, each first supporting area and a supporting structure for supporting the label area are located at a same horizontal position. Each second supporting area is lower than the first supporting area and is obliquely arranged in a gradual decrease way. "A plurality of" may be two or more.

In some embodiments, the plurality of supporting structures with inclined planes or slopes are located in a liquid diversion area. After entering the liquid diversion area, liquid flows to the sample application areas of test strips, respectively. In other words, the plurality of supporting structures with inclined planes or slopes according to the invention, for supporting the sample application area, are located in one liquid diversion area into which liquid enters. In the liquid diversion area, liquid flows to each of the testing elements. In some embodiments, a liquid diversion element is further provided in the liquid diversion area and covers the sample application area. In some embodiments, the liquid diversion element covers the sample application area located on a second inclined supporting structure.

In some embodiments, in order to cover the surface of the second supporting structure with part of the sample application area of the testing element, one press strip is arranged on a corresponding upper card and can be applied to the part of the sample application area to cover the surface of the second supporting structure. Similarly, the liquid diversion element, if any, is also allowed to cover the sample application area. It can be understood that a plurality of supporting structures with inclined planes are provided in the liquid diversion area, and the plurality of supporting structures are obliquely arranged in the liquid diversion area, and the part of the sample application area of the lateral flow test strip covers the upper surface of the supporting area of each of the inclined planes. In this way, in case of two inclined planes, the sample application areas of two test strips are covered. In case of three inclined planes, the sample application areas of three testing elements are covered. In this way, the fluid sample contacts with the sample application area on the inclined plane, flows upward to the sample application area on the surface of the first supporting structure by virtue of the capillary force of the sample application area, flows to the supporting structure for supporting the label area at a same horizontal plane as the first supporting structure, and then flows to the testing area. In this way, when the liquid diversion element covers the sample application area located on the inclined plane, liquid from the liquid diversion element flows to the sample application areas on respective test strips, so that each of the testing elements has a partial liquid sample and multiple tests can be completed by adding a same liquid sample.

In some embodiments, in order to make fluid flow smoothly and evenly to each of the plurality of testing elements, a plurality of diversion strips are arranged on the surface of the liquid diversion element, and the diversion strips enable fluid to flow to the sample application area of the testing element along them. In some embodiments, the diversion strips are arranged on the upper card. In some embodiments, the upper card is provided with a sample application hole, and the sample application hole is arranged on the liquid diversion element. The sample application hole is located in the center of the inclined supporting structure. In some embodiments, the diversion strips are distributed on two sides of the sample application hole. The diversion strips are convex ribs arranged on the inner surface of the upper card. The convex ribs are used to guide the flow of liquid and are applied to the liquid diversion element, so that the liquid diversion element is fixed in the liquid diversion area.

In some embodiments, a positioning pin is arranged near the diversion strip of the upper card, and the positioning pin is applied to the surface of the liquid diversion element, so that the part of the sample application area of the testing element abuts against the surface of the inclined supporting structure, and the lower surface of the liquid diversion element abuts against the surface of the sample application area. In this way, the part of the sample application area of the test strip is gradually lowered relative to other areas, for example, the label area and the testing area. In this way, the liquid sample is applied to the gradually lowered position or the liquid sample guided by the liquid diversion element flows from a low position to a high position, and then flows in parallel. This not only increases the testing sensitivity, but also enables the liquid sample to have a delayed flow effect, providing sufficient time for liquid to dissolve a dry label in the label area. In some embodiments, the positioning pin is arranged on a front side of the diversion strip and above the sample application area. When the upper card is installed on the lower card, the positioning pin is applied to the liquid diversion element so that the liquid diversion element is fixed onto the part of the sample application area, and the part of the sample application area covers the surface of the gradually inclined supporting structure.

In some embodiments, the sample diversion area is located in a rectangular area, and the inclined supporting structure for supporting the part of the sample application area is located on two sides of the rectangular area, respectively. In some embodiments, the inclined supporting structure for supporting the part of the sample application area is located on two sides of a short edge of the rectangular area. In some embodiments, a limiting structure is arranged on two sides of the first supporting area to limit the side-to-side movement of the test strip. In some embodiments, the rectangular area has three protruding enclosures; and the enclosures form the liquid diversion area and include the inclined supporting structure for supporting the part of the sample application area. In some embodiments, the limiting structure is located on the connecting line of another long edge of the rectangular area. In some embodiments, the short edge is provided with a plurality of protrusions contacting with the edge of the sample application area of the testing element.

In some embodiments, the testing element includes a testing area, a label area, and a liquid sample application area, where antibodies or antibody fragments are fixed on a nitrocellulose membrane in the testing area. The label area includes a label pad, the label pad is provided with antibodies or antibody fragments, and the antibodies or antibody fragments are conjugated with colored particles. In some embodiments, one end of the label pad is overlapped with one end of the nitrocellulose membrane. In some embodiments, the label pad and the nitrocellulose membrane are further provided with a blank pad, and a reagent for improving fluidity is treated on the blank pad. In some embodiments, the sample application pad is located at a tail end of the testing element, and a water absorbent pad is located at the other end of the testing element. In some embodiments, the part of the sample application pad covers the inclined supporting structure. In some embodiments, an included angle between a surface of the inclined supporting structure and a bottom of the lower card is 20-30 degrees.

### Beneficial effect

[1] The above structure can reduce the impact of excessive liquid samples on the accuracy of test results and obtain the test results quickly, which is especially suitable for women at home to self-test.

### BRIEF DESCRIPTION OF THE DRAWINGS

FIG. 1 is a structural top view of a testing element in a housing of a test device according to an embodiment of the invention.
FIG. 2 is a schematic diagram showing a three-dimensional structure of a testing element according to the invention.
FIG. 3A is a schematic diagram showing an exploded structure of two liquid diversion elements for testing different analytes according to an embodiment of the invention.
FIG. 3B is a schematic diagram showing a combination structure of two liquid diversion elements for testing different analytes according to an embodiment of the invention.
FIG. 4 is a schematic diagram showing a testing element and a supporting structure for supporting the testing element according to an embodiment of the invention (part of a sample application pad is located on but not covers a slope).
FIG. 5 is a schematic diagram showing a testing element and a supporting structure for supporting the testing element according to an embodiment of the invention (part of a sample application pad covers a slope, and a liquid diversion element is also located on the slope and covers the part of the sample application pad).
FIG. 6 is a schematic diagram showing a structure of a lower card of a test device according to an embodiment of the invention.

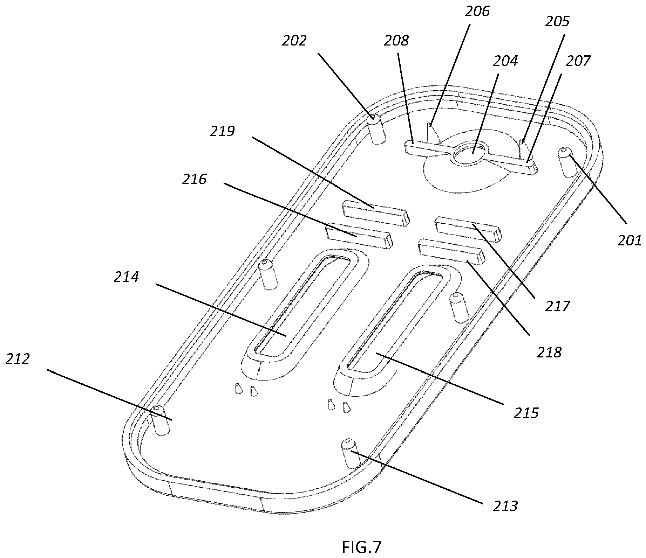

FIG. 7 is a schematic diagram showing a structure of an upper card of a test device according to an embodiment of the invention.
FIG. 8 is a schematic diagram showing a partially enlarged structure of an upper housing in FIG. 6 according to an embodiment of the invention.
FIG. 9 is a schematic diagram showing an exploded structure of a test device, a test strip, and a liquid diversion element according to an embodiment of the invention.
FIG. 10 is a schematic diagram showing a structure where a testing element and a liquid diversion element are located on a lower card according to an embodiment of the invention.
FIG. 11 is a schematic diagram showing a structure of a lower card of a test device according to another embodiment of the invention.
FIG. 12 is a schematic diagram showing a structure of an upper card of a test device according to another embodiment of the invention.

### DETAILED DESCRIPTION OF THE INVENTION

The following further describes the structures involved in the invention or the technical terms used therein. Unless otherwise specified, they shall be understood and explained according to the general terms commonly used in the prior art.

### Detection

Detection means assaying or testing presence or absence of a substance or material, including but not limited to, chemical substance, organic compound, inorganic compound, metabolite, drug, drug metabolite, organic tissue, metabolite of organic tissue, nucleic acid, protein or polymer. In addition, detection means that the amount of a substance or material is tested. Further, assay also means immunoassay, chemical assay, enzyme assay, and the like.

### Sample

The samples detected by the test device of the invention include biological liquid (for example, case liquid or clinical sample). Liquid samples or fluid samples may be derived from solid or semi-solid samples, including feces, biological tissues and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine, and preferably, the biological sample is saliva. Food samples include food processed materials, final products, meat, cheese, wine, milk, and drinking water. Plant samples include samples derived from any plants, plant tissues, plant cell cultures, and media. "Environmental samples" include samples derived from the environment (e.g., liquid samples from lakes or other bodies of water, sewage samples, earthen samples, groundwater, seawater, and waste liquid samples). The environmental sample may further include sewage or other waste water.

An appropriate test device according to the invention can be used to test any analyte. Preferably, the test device of the invention is used to test small drug molecules in saliva and urine. Of course, the samples detected by the test device of the invention may be any samples of the above forms, regardless of being solid or liquid at the beginning, provided that these liquids or liquid samples can be absorbed by the sample application area of the testing element. Generally, the sample application area is made of a water absorbent material, and liquid samples or fluid samples can be absorbed by the capillary or other characteristics of the material of an absorption element, so that the liquid sample can flow in the sample application area. The material of the sample application area may be any material capable of absorbing liquid, such as sponge, filter paper, polyester fiber, gel, non-woven fabric, cotton, polyester film, and yarn. Of course, the sample application area may be made of a water absorbent material or a non-water absorbent material. However, the absorption element is provided with holes, screw threads, and caves on which the samples can be collected. Generally, the samples are solid or semi-solid samples, and filled between screw threads and in the holes or caves for collection. Of course, optionally, the sample application area may be composed of some non-absorbent fibers and hairs, and these materials are used to scrape a solid, semi-solid or liquid sample, so that these samples can be retained on the sample application area. If detection is needed, a buffer solution is applied to the sample application area to dissolve the sample, so that the dissolved sample flows on the testing element or the detection element.

In some embodiments, the liquid sample is in contact with the diversion element, instead of being manually applied to the sample application area of the testing element of the invention, so that the liquid sample diverted by the diversion element flows to the sample application area and the label area sequentially, followed by being assayed or tested. The following further gives a detailed description with reference to the specific embodiments.

### Downstream and upstream

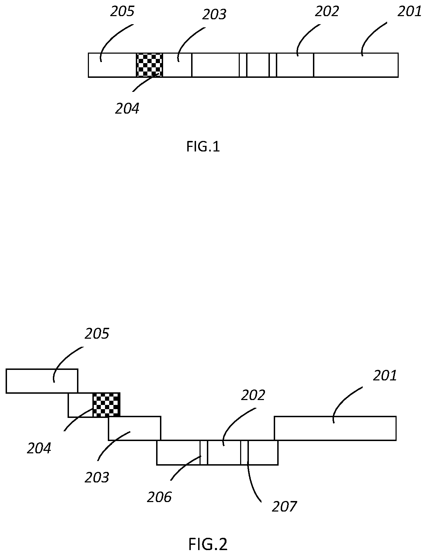

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the test device of the invention, after the liquid sample is received by the liquid diversion element, liquid can flow from the liquid diversion element to the sample application areas or the sample application pads of two testing elements, and then liquid flowing to the sample application pads flows to a downstream label pad and is mixed with the marked label, and flows to a downstream testing pad through a transition pad, where the testing area is located upstream of a test result control area on the testing pad and finally flows to a sample absorption pad on a downstream sample absorption area. The testing area may be a polyester fiber film, and the diversion element may be a glass fiber, a polyester chip, and a polyester film. In this case, the diversion element is located at the upstream of the label area of the testing element. The specific structure of the testing element is a structure 20 as shown in FIG. 1 to FIG. 2. When part of the sample application pad is located on an inclined plane and is lower than the label pad, liquid on the sample application pad flows from bottom to top mainly by virtue of the capillary force, while liquid on the surface of the sample application pad may flow downward due to gravity.

### Gas flow or liquid flow

Gas flow or liquid flow means that liquid or gas can flow from one place to another place. In a flow process, the liquid or gas may pass through some physical structures to play a guiding role. The "passing through some physical structures" here means that liquid passes through the surface of these physical structures or their internal space and flows to another place passively or actively, where passivity is usually caused by external forces, such as the flow of the capillary action and air pressure action. The flow here may also be a flow due to self-action (gravity or pressure) of the liquid or gas, and also may be a passive flow. The fluid under the action of air pressure may be a forward flow, or also a reverse flow; or a fluid is urged to flow to another position from a position under the action of air pressure. Here, the flow does not mean that a liquid or a gas is necessarily present, but indicates a relationship or state between two objects under some circumstances. In case of presence of liquid, it can flow from one object to another. Here it means the state in which two objects are connected. In contrast, if there is no gas flow or liquid flow state between two objects, and liquid exists in or above one object but cannot flow into or on another object, it is a non-flow, non-liquid or non-gas flow state.

### Testing element

The "testing element" used herein refers to an element that can be used to detect whether a fluid sample or a fluid specimen (a liquid sample or a liquid specimen) contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the invention. In the invention, the testing element and the "lateral flow testing element, or test strip" can be used interchangeably, indicating a same meaning.

Various testing elements can be combined for use in the invention. One form of the testing elements is a test strip. The test strips used for analyzing the analyte (such as drugs or metabolites that show physical conditions) in samples can be of various forms such as immunoassay or chemical analysis. The analysis mode of non-competition law or competition law can be applied for test strips. A test strip generally contains a water absorbent material that has a sample application area, a reagent area, and a testing area. Fluid or liquid samples are added to the sample application area and flow to the reagent area through capillary action. If analyteexists in the reagent area, samples will bind to the reagent. Then, samples continue to flow to the testing area. Other reagents such as molecules that specifically bind to analyte are immobilized on the testing area. These reagents react with the analyte (if any) in the sample and bind to the analyte in this area, or bind to a reagent in the reagent area. Label used to display the detection signal exists in the reagent area or the detached label area.

Typical non-competition law analysis mode: if a sample contains analyte, a signal will be generated; and if not, no signal will be generated. Competition law: if no analyte exists in the sample, a signal will be generated; and if analyte exists, no signal will be generated.

The testing element can be a test strip, which can be water absorbent material or non-water absorbent material. The test strip can contain several materials used for delivery of liquid samples. A material of the test strip can cover the other material thereof. For example, the filter paper covers the nitrocellulose membrane. One or more materials may be used in one area of the test strip, and one or more other different materials may be used in the other area thereof. The test strip can stick to a certain support or on a hard surface for improving the strength of holding the test strip.

Analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal can be in the sample application area, the reagent area or the testing area, or on the whole test strip, and one or more materials of the test strip can be filled with this substance. The solution containing a signifier is added onto the surface of the test strip, or one or more materials of the test strip is or are immersed in a signifier-containing solution. The test strip containing the signifier solution is made dry.

Various areas of the test strip can be disposed as follows: sample application area 205, label area 204, and testing area 202, where the testing area includes a test result area 206 and a test result control area 207. The control area is located behind or downstream of the testing area. All areas can be disposed on a test strip that is only made of one material. Alternatively, different areas may be made of different materials. Each area can be in direct contact with the liquid sample, or different areas are disposed according to the flow direction of liquid sample; and a tail end of each area is connected and in superimposed with the front end of the other area. Materials used can be those with good water absorption such as filter papers, glass fibers or nitrocellulose membranes. The test strip can also be in other forms.

The nitrocellulose membrane test strip is commonly used, that is, the testing area includes a nitrocellulose membrane (NC) on which a specific binding molecule is immobilized to display the test result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with the test strips disclosed in the above patents may be applied to the testing element or test device of the invention for the detection of an analyte, for example, the detection of an analyte in a sample.

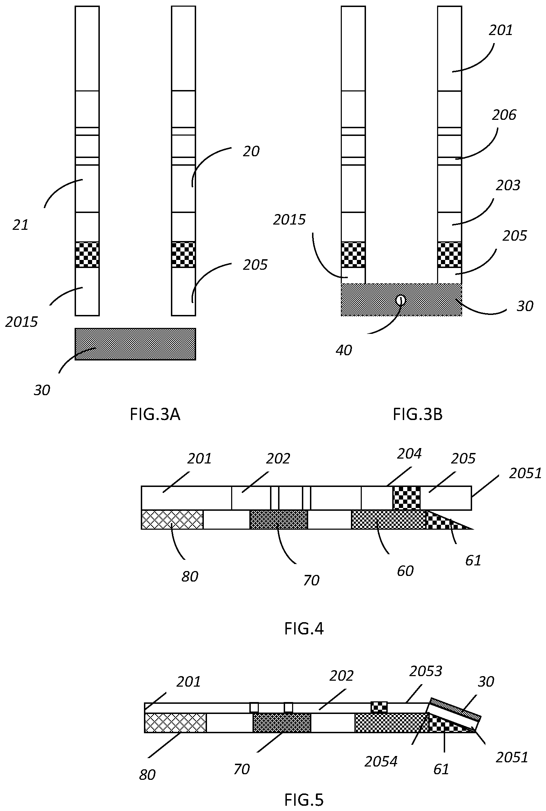

Test strips used in the invention may be commonly referred as lateral flow test strips. The specific structure and detection principle of the test strips are well known to a person skilled in the art in the prior art. A common test strip (FIG. 1 - FIG. 2) includes a sample application area 205, a label area 204, and a testing area 202. A sample collection area includes a sample receiving pad or a sample application pad, and the label area includes a label pad. The test strip may further include a water absorption area 201 to absorb the liquid sample from the nitrocellulose membrane, and the water absorption area may include a water absorbent pad. In some embodiments, the label area includes colored particles conjugated with antibodies, and the colored particles may be latex particles, gold particles, or dye particles. The testing area 202 includes necessary chemical substances for detecting the presence or absence of analyte, such as immunoreagents or enzyme chemical reagents. The sample application area or sample application pad 205 of the invention does not directly receive the sample, but is in contact with the liquid diversion element 30 to receive the liquid sample diverted by it. The part of the sample application area of the invention is covered by the liquid diversion element 30 (as shown in FIG. 3B). In some embodiments, as shown in FIG. 2A, a transition pad 203 is further arranged between the label pad of the test strip and the testing pad thereof, and the transition pad is used to delay the flow rate of the liquid on the label pad to the testing pad, so that the liquid sample can be in contact with the dry label on the label pad for a longer time, to obtain more sufficient reaction or allow the dry label to be fully dissolved by the liquid sample. The nitrocellulose membrane test strip is commonly used, that is, the testing area 202 includes a nitrocellulose membrane, and an area 206 (T-line) on which a specific binding molecule is immobilized to display the test result; and other test strips such as cellulose acetate membrane or nylon membrane test strips can also be used. Of course, in the downstream of the testing area, there may also be a test result control area 207 (C-line); generally, test strips appear on the test result control area and the testing area in the form of a horizontal line, namely, a test line or a control line. Such test strips are conventional. Of course, they can also be other types of test strips using capillary action for detection. In addition, there are dry chemical reagent components on common test strips, for example, an immobilized antibody or other reagents. When the test strip meets liquid, the liquid flows along the test strip with a capillary action, and the dry reagent components are dissolved in the liquid and treated in a next area, and the dry reagents react in the area for necessary detection. The liquid flow mainly relies on a capillary action. Here, all of the test strips can be applied to the test device of the invention or can be disposed in contact with the liquid samples in a detection chamber or used to detect the presence or absence of analyte in the liquid samples that enter a detection chamber, or the quantity thereof.

In addition to the foregoing test strip or lateral flow test strip which is used to contact with the liquid sample to test whether the liquid samples contain analytes. The testing element of the invention may be used as a test device by itself to detect an analyte in a sample. Therefore, the test device here is equal to a testing element. For example, after being mixed with a treatment solution, the fluid sample is detected with a testing element directly, specifically described as follows: When a receiving device is described to treat a fluid sample, the testing element may be used for detection alone.

### Analyte

Examples that can use an analyte related to the invention comprise small-molecule substance, including drugs (such as drug of abuse). "Drug of Abuse" (DOA) refers to using a drug (playing a role of paralyzing the nerves usually) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The detection device of the invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the pre invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the invention. The above detection way can also be used to detect the presence or absence of viral antigens, for example, COVID-19 antigen and influenza antigen.

### Housing including testing element

In some specific embodiments, the testing element may be also disposed on some carrier elements; the carrier elements include the testing element to complete the detection and assay of the analytes in fluid samples. Therefore, in some embodiments, the test device includes a carrier, and the carrier is provided with a testing element. In some embodiments, the carrier of the invention is a housing used for bearing or accommodating the testing element; the carrier element does not participate in the detection directly by itself, but serves as a carrier or housing used for bearing or accommodating the testing element. In some embodiments, the housing or carrier of the invention includes a groove structure, and the structure is used for limiting the position of the testing element on the carrier. One or more groove structures here may be available. Each groove is provided with a testing element, and the testing element may be used for testing the number or presence of analytes in a sample.

In some embodiments, FIG. 4 and FIG. 5 are a schematic diagram showing a principle structure of a test device according to the invention, and the test device includes a testing element 20 located on a supporting structure, and the supporting structure is generally located on a housing, for example, a lower housing. The supporting structure has a supporting area for supporting the sample application area 205 of the test strip. Generally, the sample application area is a sample application pad 2051 made of glass fiber, and the supporting structure for supporting the sample application pad includes a first supporting area 60 and a second supporting area 61 configured together to support an entire sample application pad 2051, where the second supporting area gradually inclines along one end of the first supporting area to form a section of the inclined supporting area 61 with gradually lowered height, and the surface of the inclined supporting area covers part of the sample application pad 2052. In this way, the entire sample application pad is divided into two parts by the sample supporting structure, one part thereof covers the surface of the inclined supporting area 61. The other part thereof covers the first supporting area 60. The first supporting area is also covered by the label pad and the part of the sample application area 2052, so the label pad, the part of the sample application pad, and the testing pad are in a same horizontal plane, and the part of the sample application pad is located on a slope. Of course, the sample application pad described here is generally bonded to a non-water absorbent liner, so the sample application pad indirectly covers the surface of the second supporting structure through the liner, instead of indirectly covering it. Similarly, nitrocellulose as the testing pad, the label pad with a label, and the water absorbent pad can be bonded to the non-water absorbent liner. In some embodiments, when it is desired that a plurality of testing elements can be tested at the same time by applying samples once, the liquid diversion element 30 is further provided and covers parts of the sample application areas 2015, 205 of the first testing element 20 and the second testing element 21, so that the liquid diversion element is connected with the two testing elements to form fluid communication between them. In some embodiments, the liquid diversion element covers the inclined sample application areas of the two testing elements (as shown in FIG. 5). It can also be understood that the liquid diversion element 30 also covers the surface of the inclined second supporting area 61 by covering the surface of the sample application area 2052, instead of directly covering it. The two testing elements are arranged in parallel when connected, and the sample application areas covered by the liquid diversion element 30 are respectively located in the inclined second supporting area. It is easy to understand that when two test strips are used, two supporting structures for supporting the test strips are arranged in parallel and include the inclined supporting structure for supporting the part of the sample application pad. In some embodiments, a sample receiving area 40 is arranged in the center of the liquid diversion area, and the liquid sample can be directly applied to the sample receiving area of the liquid diversion element, so that the liquid sample flows from two sides of the liquid diversion element to the two testing elements for testing different analytes (FIG. 3B).

In conventional devices like this, a common problem here is that capillary fibers used to make up the liquid diversion element are not evenly distributed due to the material problem of the liquid diversion element, when liquid is conveyed by virtue of the capillary force, there is naturally a difference in flow rate and the thickness of the liquid diversion element is not uniform, so that liquid is applied to the sample application area 40, and liquid flowing to the two testing elements is not evenly distributed; for example, when 1ml of the liquid sample is applied, only 0.2 ml thereof may flow to the testing element 20 and the other 0.8 ml thereof flows to the testing element 21 (in some extreme cases), so that liquid on the testing element 20 is not enough to complete the entire test; if there are excessive liquid samples on the testing element 21, this also affects the final test result, for example, flooding phenomenon. Therefore, in order to overcome the problem of uneven distribution of liquid samples, it is found that the arrangement of the inclined structure can significantly mitigate the problem of uneven distribution. In addition, there is a specific distance between the two testing elements, and the liquid sample receiving area is directly located on the liquid diversion element, but not directly located on the sample application areas 2015, 205 of the testing elements. When applied to the sample receiving area 40 of the liquid diversion element, liquid flows from the center to two sides, and liquid on the inclined plane flows upward. If there is too much liquid, such liquid flows from the center to the area below the liquid diversion element without excessively flowing onto the test strip, and at the same time, an inclined supporting structure is arranged obliquely, so that the flow rate of the liquid on the liquid diversion element slows down due to the gravity of the liquid itself, and an amount of liquid flowing to the sample application area of the test strip can be kept stable. Here, the sample application area of the test strip is shorter than that of a common test strip, and an amount of the liquid needed is relatively small, and the liquid flowing to the sample application pad quickly flows to the label pad, so that excessive samples are not required to flow to the testing element and the liquid sample required for each test strip is about 10ml-50ml. Generally, the sample application pad is an integral component; an inclined plane being provided is equivalent to folding of the sample application pad. For example, at a folding location 2054, the part of the sample application pad 2052 is fixed on the inclined plane, and the sample application pad is pressed in a downstream area of the folding location 2054, and a folded structure also slows down the flow of liquid, so that folding and arrangement of the slope lead to slowing down the overall rate of the liquid when there is a sufficient amount of the liquid sample and the length of the sample application pad is short, and the flow rate of the liquid is kept consistent in a very short distance, thereby providing more time for the liquid to contact and dissolve a dry downstream label, and making the analyte of the sample react with the label to increase the sensitivity.

In some embodiments, in order to make liquid dripped on the sample receiving area 40 of the liquid diversion element 30 flow to the testing elements connected to two sides more quickly, a diversion strip is further arranged on the liquid diversion element. When the diversion strip is pressed on the surface of the liquid diversion element 30, the liquid diversion element is attached only to the sample application pad 2052 of the testing element, and at the same time, the sample application pad is attached only to an inclined supporting surface. The diversion strip has no water absorption, for example, some plastic strips. However, a capillary structure is formed when the diversion strip contacts with the surface of the liquid diversion element. By virtue of the capillary structure, the liquid sample from the sample receiving area of the liquid diversion element quickly flow to the test strips 21, 20 distributed on two sides. These diversion strips can be located on the upper card; a sample application hole is provided on the upper card and located on the sample receiving area 40 of the liquid diversion element 30, and the diversion strips can be distributed on two sides of the sample application hole of the upper card and can also be located on the upper card. When the upper card is combined with the lower card, the sample application hole covers the sample receiving area 40 of the liquid diversion element, and the diversion strips distributed on two sides of the sample application hole are pressed on the surface of the liquid diversion element 30.

Sample application pads, label pads, transition pads, testing pads, and water absorbent pads of common test strips are bonded to a rigid liner card. The liner card has certain rigidity and is vertical in a normal state. The above pads are water-absorbent and have thin walls, so part of the sample application area located on the liner is easy to return to the original straight state (as shown in FIG. 4) when bent. In the invention, it is always desired that part of the sample application area or the sample application pad is bent to cover the surface of the supporting structure 61. In order to achieve the object, a protruding fixing pin is provided on the inner surface of the upper housing, and the pin protrudes from the inner surface of the upper card. Then, when the upper card is combined with the lower card, the pin can be contacted in a length range from the inner surface to the center of the inclined plane (shown by an arrow in FIG. 5), so that the fixing pin can be applied to the liquid diversion element, and additionally applied to the sample absorption pad, and part of the sample absorption pad is closely attached to the inclined plane of the supporting area 61. The part of the sample application area 2052 of the testing element is always kept at an inclined angle relative to the position of the other part of the sample application pad 2053. Alternatively, the sample application pad is bent to form two parts. The fixing pin is a simplest way to achieve such bending. Of course, glue is also applied to the inclined plane, so that the part of the sample application pad 2053 is bonded to the surface of the inclined plane and the glue is also applied to the surface of the supporting area 61. In such a way, bending can also be formed, but a bending process is very complex. This is not conducive to automatic production.

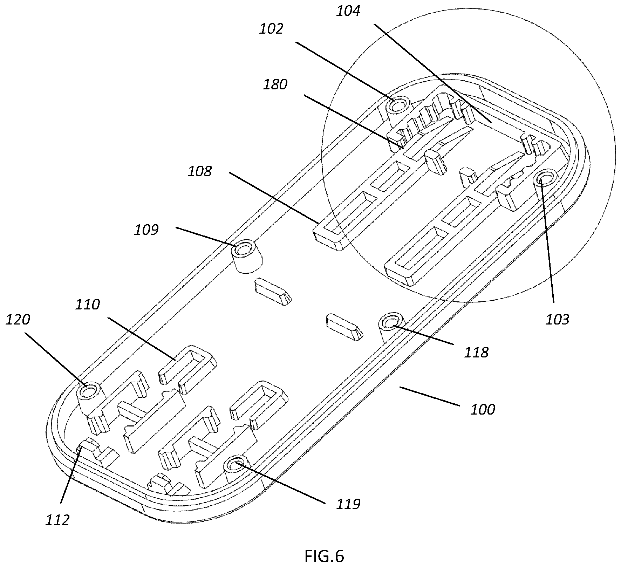

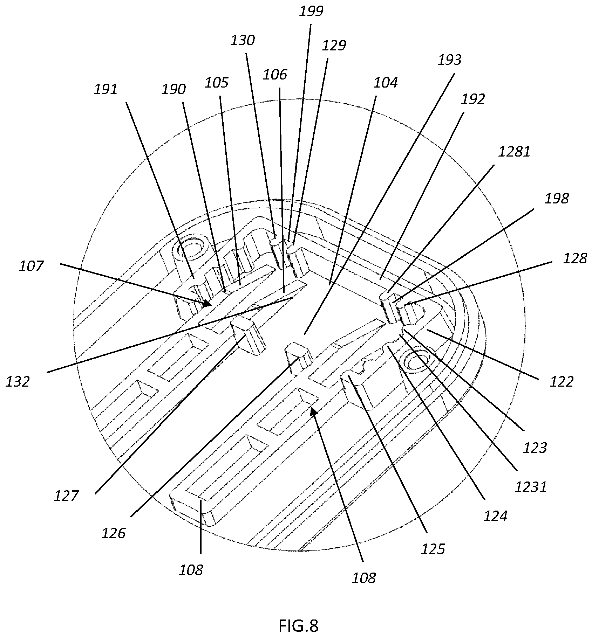

In some embodiments, the invention provides a device, and the device includes an upper housing 100 and a lower housing 200. The lower housing 200 includes a supporting structure for supporting lateral flow testing elements 21, 20, and the supporting structure is used for placing the testing elements. The supporting structure includes a supporting structure unit 110 for supporting a water absorbent paper, a supporting structure unit 117 for supporting a testing pad, a supporting structure unit 108 for supporting a label pad, and a supporting structure unit 180 for supporting a sample application pad. In some embodiments, parts of the supporting structures 106, 105 for supporting the sample application pads are located lower than the supporting structure 107 for supporting the other parts of the sample application pads. It can also be considered that the testing element provided includes a sample application pad 205, a label pad 2041, a testing pad 207, and a water absorbent pad 201. The sample application pad 205 is folded. In some embodiments, a tail end of the sample application pad is inclined to the folding location 2054, or a tail end of the sample application pad is gradually increased to the folding location. In some embodiments, in order to achieve the state that the part of the sample application pad is inclined, a supporting structure 180 for supporting the sample application area is arranged on the bottom of the lower card and partially inclined, so that the part of the sample application pad covers the surface of the inclined supporting structure, and the part of the sample application pad is also inclined. As shown in FIG. 6, the supporting structure includes two supporting strips 107, 180, arranged symmetrically and in parallel, and a tail end of each of the supporting strips is provided with inclined planes 106,105. The inclined surfaces 106, 105 have a same angle and are arranged in parallel. When the lateral flow test strip is placed on the supporting structure, one end thereof with the sample application area is in contact with a pair of limiting structures 128, 129, while a tail end thereof with the water absorbent pad is in contact with a limiting structure 112. In this way, the length of the entire test strip is determined as a distance between the protruding limiting structures 129, 112. In this case, an entire test strip is located on the supporting structure, and the water absorbent pad is located on the supporting structure 110 of the water absorption area. The testing pad, for example, a nitrocellulose membrane area, is located on the supporting structure 117 for supporting the testing pad, while a label pad area and a sample application pad area are both located on the supporting structure 108. A tail end of the supporting structure is arranged obliquely. In other words, it is desired that the part of the sample application pad covers the inclined planes 105, 106 of the supporting structure. In some embodiments, the lengths of the entire inclined planes 106,105 are about 1/3 or 1/2 of the length of the entire sample application pad. In this way, the part of the sample application pad of the testing element and the label pad are located in a same horizontal plane, the sample application pad at the tail end is located in the inclined plane, and a folding location 190 is provided in a plane where the inclined plane and the label pad are located. In some embodiments, the sample application pad in the inclined plane is used to receive liquid samples. The so-called sample application pad here is used for receiving liquid samples instead of directly applying samples, for example, receiving samples from the liquid diversion element, instead of directly receiving applied samples. As shown in FIG. 6 and FIG. 8, two inclined supporting structures and a pair of supporting structures for supporting the test strip are arranged in the lower card. In this way, two test strips can be placed in the lower card at the same time; and each of the test strips can be used to test different analytes, for example, one of the test strips is used to test COVID-19 antigen and the other thereof is used to test influenza antigen. However, a plurality of parallel supporting structures may be provided and can be used for placing test strips. The sample application pads or the sample receiving pads of the test strips are covered by the liquid diversion element and are in fluid communication with each other. In some embodiments, the lower card is provided with one liquid sample diversion area 104; the liquid sample diversion area is a rectangular area and surrounded by three enclosures 192, 122, 191 protruding upward from the bottom 193 of the lower card, and the enclosure is not provided on a side opposite the enclosure 192, so that the supporting structure for supporting the sample application area of the test strip extends into the liquid sample diversion area 104. In addition, the inclined supporting structure, including a pair of parallel inclined planes 105, 106, is located in the sample diversion area 104, and a tail end of the inclined plane is located nearby the limiting structure 129, and the limiting structure is formed by a pair of protruding structures 129, 130 with an outward inner wall of the enclosure 192, and these structures can limit the tail end of the sample application pad, and a groove formed between the protruding structures can discharge liquid. In some embodiments, an inclined angle 132 is 25-35 degrees, indicating the height of the inclined plane.

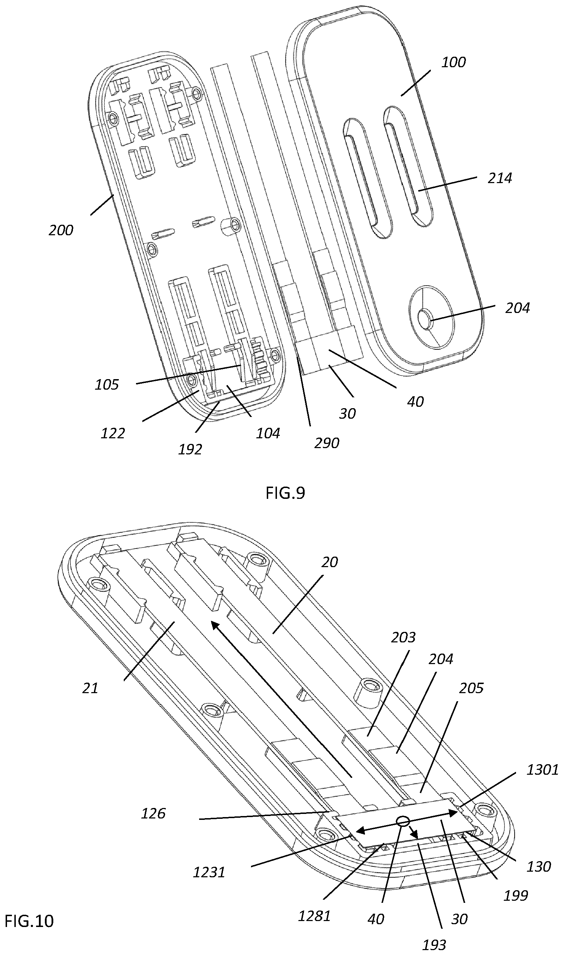

In some embodiments, when the two test strips are placed on the supporting structure, one liquid diversion element 30 is arranged at tail ends of the sample application pads of the two test strips, and the size of the liquid diversion element 30 is equivalent to that of the liquid sample diversion area 104 (as shown in FIG. 10), so that the liquid diversion element covers parts of the sample application pads of the two testing elements. In particular, the width of the liquid diversion element is the same as the length of the inclined planes 106, 105, so that part of the sample application pad can cover the inclined planes 106, 105 when a specific pressure is applied to the liquid diversion element, thereby making the liquid diversion element and the part of the sample application pad form an inclined angle. It can be seen from FIG. 8 that the test strips are distributed on two sides close to the liquid sample diversion area 104, and the two test strips have a specific distance in the liquid sample diversion area and are not close together. In some embodiments, the liquid diversion element is provided with one sample receiving area 40, and the sample receiving area 40 is located in the center of the two test strips, and can also be considered to be located in the center of the liquid diversion element. In this way, viewed from a physical structure, liquid from the liquid diversion element can flow to the two testing elements, and it is desired that the volume of the liquid flowing to each of the test strips is equal. In some embodiments, the sample receiving area of the liquid diversion element corresponds to a sample application hole 204 of an upper housing 200, so that applied liquid sample flows directly to the sample receiving area 40 of the liquid diversion element, and then is diverted into the two test strips through the sample application pad. The sample receiving area 40 on the liquid diversion element 30 is located in the center, and its advantages are mainly reflected in the following aspects: it can be seen from FIG. 8 that a blank area is present between the two test strips, and a low-lying place 193 is relatively formed in the liquid sample diversion area 104 due to the action of the supporting structure. If an amount of the sample applied through the sample application hole 204 of the upper card is relatively large, an excess of the sample flows to the low-lying place 193 along the inclined liquid diversion element for collection, liquid in the low-lying area is far away from the test strips and does not flow thereto, and flooding caused by a large amount of liquid flowing to the sample application pad is reduced due to the blocking action of the protruding supporting structure. This is because the liquid diversion element is also rectangular and inclined, the liquid receiving area is located in the center, part of the liquid flows laterally (as indicated by a lateral arrow in FIG. 10) to the testing elements on two sides relative to the lower card under the capillary action of the liquid diversion element, while an excess of liquid flows directly to the low-lying area along the longitudinal direction of the liquid diversion element (along the longitudinal direction of the lower card) (as indicated by an arrow in FIG. 10), instead of flowing to the test strip. As the liquid diversion element is rectangular, the distance of liquid flowing directly to the testing element is long, but the distance of liquid flowing to the low-lying place is short. This helps the excess of liquid flow, so that the lateral flow mainly depends on the capillary force. It is mainly considered that these test devices are operated by inexperienced persons. The most common problem is that liquid is excessively applied. Originally, 2-3 drops of liquid, about 40µL-30µL, are needed, but 50µL-500µL liquid is often applied, and sometimes 1 ml of liquid is applied. Through such arrangement, the excess of sample flows to the low-lying place in the liquid sample diversion area 104, and the normal sample flows to the inclined sample application areas on the lateral flow testing elements arranged at two sides. The test device of the invention is greatly improved as compared with a conventional test device for testing multiple analytes with one sample. In addition, an appropriate amount of sample, if any, flows from the sample receiving area 40 to two sides along the liquid diversion element and respectively flow to the sample application areas of the two test strips. In addition, parts of the sample application areas of the test strips are located at an inclined angle, and the other parts thereof, the label pad, and other testing pads are located at a same horizontal position, while the inclined sample application pad is located at a low position and the label pad is located at a high position. The liquid sample actually flows from a low position to a high position when flowing to the inclined sample application pad. When the sample application pad is very short, it is desired that the liquid flows slowly on the test strip. Normally, the liquid flows slowly from the low position to the high position under gravity. In this way, the liquid has more time to contact with a dry label when flowing to a label area. If the sample contains the analyte, the dissolved label has more time to react with the analyte so that they are more fully combined. Another advantage thereof is that: the liquid sample being excessively diverted from the liquid diversion element 30 to the sample receiving area often occurs in actual operation, and the liquid diversion element is only 10mm-12mm long and about 1mm-2mm thick, so saturated liquid is about 10µL-15µL; excessive liquid, if any, flows to the sample receiving area, and flows from the surface of the sample receiving area to the lower position at the inclined angle (under gravity), instead of flowing to the high position, and the liquid flowing to the high position completely depends on the capillary force. When the liquid flows to some water absorbent materials with capillary action, it is generally desired that the liquid moves forward under the capillary action, which is the basic principle and property of a lateral flow test strip. However, if there is too much liquid, part of the liquid moves from upstream to downstream under the capillary action, but the other part of the liquid on the surfaces of the water absorbent materials (the capillary has been completely moistened) naturally flow. In this case, the flow rate of the liquid is faster than that of the liquid under the capillary action. In actual detection, such problem occurs if the sample is excessively applied, which leads to that the liquid on the surfaces of the water absorbent materials reaches the sample application area in advance, moistened in advance are treated specifically on the testing area of the testing pad, for example, if antibodies or antigens are moistened in advance, the capillary force is lost, and subsequent labels or label mixtures lose capillary actions thereof and continue to move or move to the testing area, causing wrong test results. For example, if the liquid flowing to the sample application area is excessive, part of the liquid may flow by virtue of the capillary force, and the other part thereof may directly and quickly flow on the surface of the sample application area (without depending on the capillary force). However, during the lateral flow test, it is desired that the liquid flows by virtue of the capillary force, instead of directly flowing on the surface of the sample application area, so that the test results are more accurate. Because the liquid quickly flows on the surface of the sample application area, some downstream areas are moistened in advance; however, liquid for dissolving dry reagents slowly flows by virtue of the capillary force, downstream areas are moistened in advance (the capillary force is lost or weakened) and subsequent reagents cannot continue to move or the motion dynamics thereof are lost, so that liquid with reagents cannot flow or gives rise to low flow and is little combined on the testing area, thereby affecting the accuracy of the test results. In fact, although the liquid diversion element is directly used to receive the liquid sample, such problem still occurs; part of the liquid sample flows by virtue of the capillary force; if an amount of the liquid sample is large, the part of the liquid sample directly flows from the surface of the liquid diversion element, the liquid flowing on the surface of the liquid diversion element faster flows than the liquid flowing by virtue of the capillary force, which leads to excessive liquid flowing to the sample application pad; in addition, excessive liquid on the sample application pad leads to inaccurate test results. According to the invention, an inclined liquid diversion element is adopted, so that excessive liquid can directly flow to a low-lying place in the liquid sample diversion area along the longitudinal direction of the lower card (as indicated by an arrow in FIG. 10). Here, the so-called "low-lying" place of the liquid sample diversion area 104 can be formed by arranging holes or recesses in the inner surface of the lower card to collect liquid, or may be formed at the bottom of the liquid diversion area because the position of the supporting structure is high relative to the inner plane, rather than being deliberately made.

In order to ensure that excessive liquid on the liquid diversion element 30 can flow to the low-lying place, protrusions are arranged on enclosures 192, 122, 191 around the liquid sample diversion area 104 and used to fix the liquid diversion element, so that the liquid diversion element does not contact with the enclosures; however, grooves 1231 are formed between two of the protrusions and actually discharge the excessive liquid into the low-lying place. For example, in FIG. 8, protrusions 125, 124, 123 are arranged on the enclosure 122 of the liquid sample diversion area, grooves are formed between each two of the protrusions, and the surfaces of the protrusions are in direct contact with the cross section of the liquid diversion element and the side 290 of the sample application area of the testing element (that is, the thick side of the testing element). On the one hand, the testing element is allowed to be fixed in different ways. On the other hand, excessive liquid (if any) flows to a low-lying place through the grooves, instead of flowing to the testing element. Such design is also used in another enclosure 191; for example, some protrusions are provided on the another enclosure, and grooves are formed between the protrusions and used to discharge excessive liquid. A similar structure is also provided at the position of the enclosure 192 that is of the liquid sample diversion area 104 and is corresponding to the tail end of the sample application area of the test strip, and has a pair of protrusions 129, 130 or protrusions 128, 1281 between which grooves 198, 199 are formed. These protrusions are to keep the testing element out of direct contact with the enclosure, and the grooves are to discharge excessive liquid from the testing element or the liquid diversion element, focusing on the excessive liquid from the liquid diversion element. In some embodiments, positioning pins 127, 126 are provided near the supporting structure; and the positioning pins being located beside the supporting structure is mainly to accurately position the position of the test strip on the supporting structure. During the lateral flow test, fitting of the upper housing and the lower housing also has a significant impact on the test specificity and sensitivity of the test strip; in particular, some structures affecting liquid flow directly affect the testing performance of the test strip.

In some embodiments, in order to enable part of the sample application area of the testing element to be fixed in an inclined state or allow the part of the sample application area to cover the surfaces 105, 106 of the inclined supporting structures through some structures of the upper card, fixing pins 206, 205 are arranged on the upper card, and the length thereof can substantially allow the fixing pins to contact with the inclined plane when the upper card and the lower card are assembled, so that the fixing pins press the liquid diversion element to allow the liquid diversion element and the part of the sample application area to be in an inclined state. The fixing pin here can be understood as the meaning of "pressing and applying pressure", that is, the fixing pin applies pressure to the liquid diversion element and some samples to cover the inclined supporting structure. This is because when the lateral flow test strip is produced, the sample application pad 205, the label pad 204, the transition pad 203 (if necessary), the testing pad 207, and the water absorbent pad 201 are generally bonded on a rigid cardboard. As above, the sample application pad is an integral component, for example, a piece of glass fiber is bonded on the cardboard, so that the test strip is placed on the supporting structure of the lower card, and the part of the sample application area abnormally covers the inclined plane (as shown in FIG. 4). When covering the parts of the sample application pads of the two testing elements, the liquid diversion element abnormally covers the inclined plane, so it is necessary to exert a specific pressure to overcome the reverse elastic force of the cardboard and allow the liquid diversion element and the part of the sample application area to be at an inclined position. In some embodiments, the diversion strips 208, 207 are arranged on two sides of the sample application hole 204 of the upper card and are protruded from the inner surface of the upper card, and the surfaces thereof are also in contact with the liquid diversion element 30 and are substantially pressed against the surface of the liquid diversion element in the direction as indicated by a lateral arrow in FIG. 10. The function of the diversion strip is to make the liquid thereon flow to the sample application area of the testing element as soon as possible, especially to the inclined sample application area.

In some embodiments, the upper card is further provided with some pressing label pads and transition pads, as well as places where the sample application pads and the label pads overlap with each other, so that liquid can smoothly flow on each of the sample application pads. The press strips being pressed at an overlapping location of the label pad 204 and the transition pad 203 can also delay the flow of the liquid, but the contact time of the liquid and the dry label pad is prolonged. This leads to an increase in reaction time. In addition, these press strips also make the testing element tightly attach to the supporting structure, so that all areas such as the label area, the part of the sample application area, and the testing area are located in a same horizontal plane. The upper card further includes windows 214, 215 for reading or observing test results of the testing pad. In some embodiments, some rivets 202, 201, 211, 210, 213, 212 are further provided in the upper card and respectively corresponding to rivet holes 102, 103, 108, 118, 119, 120 in the lower card; the rivets fit with the rivet holes so that the upper card and the lower card are assembled and fixed together. Such fixing allows the test strips to be located at the fixing positions of the upper card and the lower card; at the same time, the fixing pins 206, 205 and the diversion strips 208, 207 are combined, so that the part of the sample application area is close to the surfaces 105, 106 of the inclined supporting structure, the diversion strips are in contact with the surface of the liquid diversion element, and the sample application hole 204 is located in the center of the sample receiving area 40 of the liquid diversion element.

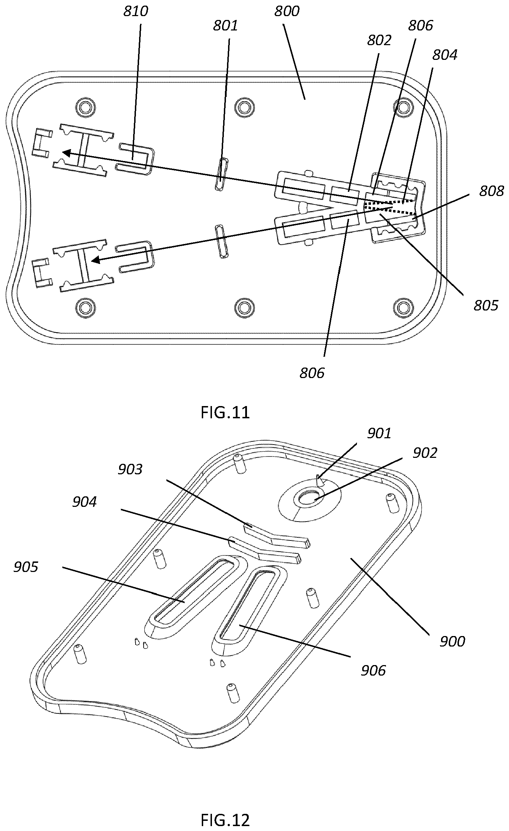

In other embodiments, the invention provides a test device, and the test device includes an upper card 800 and a lower card 900, where a supporting structure for supporting a testing element is arranged on the lower card, the supporting structure includes a supporting structure 810 for supporting a water absorbent pad, a supporting structure 801 for supporting a testing pad, a supporting structure for supporting a label pad, and a supporting structure for supporting part of a sample application pad; when two test strips are used for detection, the sample application areas thereof (the sample application area 806 of the first test strip and the sample application area 805 of the second test strip) are allowed to be overlapped in the liquid sample diversion area 808; and the supporting structures intersect with each other at a location near the sample application area, one liquid diversion area 808 is provided at an intersection location, and the liquid diversion area and the part of the sample application pad overlap in a common area 804. The common area 804 for overlapping is used to receive a liquid sample. In this way, different analytes can be tested by applying samples once. As shown in FIG. 11 and FIG. 12, a supporting structure 801 for supporting a first test strip is provided on the lower card 800, a common area 804 for supporting part of a sample application pad of a second test strip is provided on an area 803 where the supporting structure is used to support the sample application pad of the first test strip, and the common area 804 is provided with part of the sample application pad of the first test strip and part of the sample application pad of the second test strip both which are located at an overlapping location thereof. In some embodiments, the sample application pad is generally a layer of glass fiber with a thickness of 1mm-2mm (in this case, part of the sample application area is short of a liner); two layers of glass fiber (if any) overlap on the common area 804; When the upper card and the lower card are assembled, the sample application hole of the upper card is located in the center of an interaction area, and part of the sample application hole is located on the sample application pad in an overlapping area. In addition, non-overlapping sample application areas on two test strips are evenly or equally covered by the sample application hole. In this way, the liquid sample from the sample application hole can evenly contact the sample application areas of the two test strips, so that the volumes of the obtained samples are substantially equal. Similarly, a fixing pin 901 is further provided on the upper card and located in the common area 804 where the sample application pads overlap with each other, so that the sample application pads are located different fixing positions. The upper card is further provided with press strips 903, 904 to exert pressure to the overlapping location of the label pad and the sample application pad, and is further provided with two windows 906, 905 corresponding to the testing areas of the test strips.

Example 1 Fabrication of lateral flow test device for testing COVID-19 antigen and influenza A+B antigen in saliva by immunoassay provided in the invention

A lateral flow test device for testing COVID-19 antigen and influenza A+B antigen in saliva by immunoassay, fabricated in the embodiment, is as shown in FIG. 1, FIG. 3, FIG. 9, and FIG. 10; the test device includes a first test strip 21; according to a liquid flow direction, the test strip sequentially includes the sample application pad 205, the label area 204, and the testing area 206 from upstream to downstream; and the testing area and the control area 207 are located on the nitrocellulose membrane area 202, the label pad has a first antibody specifically bonding to COVID-19 antigen, conjugated gold particle, and a second antibody specifically bonding to COVID-19 antigen. Then, the transition pad 203 is located downstream of the label pad; and the transition pad and the label pad are assembled together in an overlapping mode shown in FIG. 2 to form a test strip for testing the COVID-19 antigen. The second test strip 20 for influenza test has a same structure as the first test strip, and is used only for testing the first antibody specifically binding to the influenza A+B antigen in the testing area and the second antibody specifically binding to influenza A or B antigen in the label area, and the first antibody and the second antibody are conjugated with gold particles.

Here, a length of the sample application pad of the test strip is 14mm and a width thereof is 4mm; and a width of the test strip is 4mm and a thickness thereof is 2mm. A length of the liquid diversion element is 17mm and a width thereof is 6mm, and therefore a width of the liquid diversion element covering the sample application area of each test strip is 6mm; and a width of the liquid diversion element not covering the sample application area between two test strips is 9mm, where a distance between two test strips is about 9mm. An inclined plane for supporting part of the sample application pad, arranged on the lower plate, is 6mm long. The upper card and the lower card are assembled together, so that the sample application hole is located in the center of the liquid diversion element, and the distance between the two test strips is 9mm. Such arrangement is made as shown in FIG. 10. Then, the upper card is covered to form a complete test device. A length of the liquid diversion area of the lower card is equivalent to that of the liquid diversion element, the liquid diversion area is 19mm long, 7mm wide, and 4 deep, and the supporting structure is also about 4mm high, whereby forming a low-lying place with a depth of about 4mm.

The label area includes an antibody conjugated with label particles (for example, gold particles, latex particles or dyes, or other colored labels). Then, a label mixture is sprayed on a polyester film by spraying equipment to be made into the label pad; the label on the label pad can flow along with liquid; the nitrocellulose membrane is used in the testing area, the antibody of the test line is dissolved with a buffer solution PBS, and the nitrocellulose membrane is scribed by a film-spotting device and then dried in an oven for later use. The antibody treated on the membrane usually does not move.

The mixture of COVID-19 positive sample and A+B positive saliva inactivated sample is used for testing. Lateral flow test results of these samples are all positive, and a color depth thereof is G3 (COVID-19 positive, and influenza positive) (as compared with a standard color card configured in a laboratory, the test result is positive at G3, negative at less than G3, and positive at greater than G3). A blank PBS solution is used as negative control.

Example 2: Different from Example 1, the lower card is arranged in a conventional way, a structure with a same height as a supporting structure at the label pad is arranged, instead of an inclined supporting structure. In the embodiment, except for the fixing pin, other structures are retained on the upper card. In Example 1 and Example 2, no groove is formed around the enclosure, but the groove is formed in the liquid diversion area.

Example 3: Compared with Example 1, grooves are additionally formed in three enclosures in the liquid diversion area, where the number and structure of the grooves are the same as those of the grooves shown in FIG. 8.

Testing process: in different testing groups prepared, each 20 samples are provided for devices in Example 1 and Example 2, and then standard samples prepared are used for indoor test. The testing method is as follows: for each device, respectively aspirate 40µL, 50µL, 100µL, 200µL, 500µL, and 1000µL negative sample and positive sample with a pipettor, then apply them to the sample receiving area of the liquid diversion element through the sample application hole of the upper card, and observe test results thereof after 5 minutes.

**Table 1: Results of positive sample**

| Amount of positive sample | Example 1 (Result/Number) | Example 2 (Result/Number) | Example 3 (Result/Number) |
|---|---|---|---|
| 10µL | G3/20 | G3/20 | G3/20 |
| 50µL | G3/20 | G3/20 | G3/20 |
| 100µL | G3/20 | G3/20 | G3/20 |
| 200µL | G3/20 | G3/17 (absence of T line and C line for three samples) | G3/20 |
| 500µL | G3/19 (presence of T line for all samples and absence of C line for one sample) | 3/15 (absence of T line and C line for four samples) | G3/20 |
| 1000µL | G3/19 (absence of C line for one sample) | G3/10 (absence of T line and C line for eight samples) | G3/20 |

Remarks: G3 is a positive result. In case of absence of C line, the test result is invalid. In case of absence of T line, the test result is inconsistent with the actual result.

With observation on the flow of the test devices in Embodiments 1-3, it is found that when the applied sample is more than 200ml, the number of samples without T line increases, it is observed that these samples flow to the nitrocellulose membrane to moisten the T line in advance, and liquid with colored particles is blocked, flows out of the label pad, and then contacts the nitrocellulose membrane, thereby leading to invalid test. When the liquid is 500µL, one sample has no C line with T linein Example 1. This is probably because the amount of liquid being excessively large leads to presence of the T line and absence of the C line, indicating that particle labels do not flow to the C line, which causes invalid results. In Example 2, there are four invalid results; when the sample is added to 1ml, there is still one invalid result in Example 1, but there are eight invalid results in Example 2, two of which cannot be determined. However, as compared with Example 3, a valid test can be performed when the liquid volume ranges from 10µL-1ml, indicating that an amount of the liquid can be controlled in the range of 10µL-1ml in the design and the test results are not affected by the liquid volume. This actually has significance for the OCT market for their own test, many persons not professionally trained cannot be subject to many restrictions, and at least the amount of liquid sample is not restricted, so that home self-test products have more practical value.

**Table 2: Negative control PBS solution**

| Amount of positive sample | Example 1 | Example 2 | Example 3 |
|---|---|---|---|
| 10µL | G1/20 | G2/20 | G1/20 |
| 50µL | G1/20 | G2/20 | G1/20 |
| 100µL | G1/20 | G1/20 | G1/20 |
| 200µL | G1/20 | G1/18 (absence of C line for two samples) | G1/20 |
| 500µL | G1/20 | G1/15 (absence of C line for four samples) | G2/20 |
| 1000µL | G1/20 | G1/10 (absence of C line for ten samples) | G2/20 |

It can be seen from negative results that correct results can be obtained in both Example 1 and Example 3, while a varying number of invalid results is still present in Example 2.

This specification also includes the subject matter of the following clauses:
1. A device for testing an analyte in a fluid sample, comprising: a housing comprising an upper card and a lower card, wherein a supporting structure for supporting a lateral flow testing element is arranged on the lower card, the lateral flow testing element is configured to test the analyte in the fluid sample and comprises an application pad for receiving the liquid sample, the supporting structure comprises a supporting structure for supporting the entire sample application pad, and a part of the supporting structure for supporting the sample application pad is inclined.
2. The device according to clause 1, wherein the lateral flow testing element further comprises a label pad and a testing pad, wherein the label pad is located downstream of the application pad for receiving a liquid sample, and the other part of the supporting structure for supporting the sample application pad and a supporting structure for supporting the label pad are located in a substantially horizontal position.
3. The device according to clause 2, wherein a position of the inclined supporting structure is lower than that of the supporting structure for supporting the label pad.
4. The device according to clause 3, wherein the lateral flow testing element comprises a first lateral flow testing element having a first sample receiving pad and a second lateral flow testing element having a second sample receiving pad, wherein part of the inclined structure for supporting the sample application pad comprises a first inclined supporting structure and a second inclined supporting structure; and part of the first sample receiving pad covers the first inclined supporting structure, and part of the second sample receiving pad covers the second inclined supporting structure.
5. The device according to clause 4, wherein the first inclined supporting structure and the second inclined supporting structure are spaced apart in parallel.
6. The device according to clause 5, further comprising a liquid diversion element made of a water absorbent material, wherein the liquid diversion element covers the part of the first sample receiving pad and the part of the second sample receiving pad.
7. The device according to clause 6, wherein the lower card further comprises a liquid sample diversion area, the first inclined supporting structure and the second inclined supporting structure are located in the liquid sample diversion area, and the first inclined supporting structure and the second inclined supporting structure are distributed at two ends of the liquid sample diversion area.
8. The device according to clause 7, wherein the liquid diversion element is parallel to the first inclined supporting structure and the second inclined supporting structure or also obliquely arranged.
9. The device according to clause 7, wherein a liquid sample receiving area is arranged at the center of the liquid diversion element and located between the first inclined supporting structure and the second inclined supporting structure.
10. The device according to clause 6, wherein the liquid sample diversion area comprises a low-lying area, and liquid from the liquid diversion element obliquely arranged is capable of flowing to the low-lying area for collection.
11. The device according to clause 10, wherein the upper card comprises a liquid sample dripping hole, and the liquid sample dripping hole is located in the liquid sample receiving area of the liquid diversion element.
12. The device according to clause 11, wherein two sides of a sample application hole of the upper card are further provided with a diversion strip, respectively; and the diversion strip is in contact with the surface of the liquid diversion element.
13. The device according to clause 12, wherein the diversion strip is located on the first inclined supporting structure and in contact with the liquid diversion element on the second inclined supporting structure.
14. The device according to clause 13, wherein the upper card further comprises a pressing pin, and the pressing pin is arranged on the liquid diversion elements on the first inclined supporting structure and the second inclined supporting structure, respectively, so that the liquid diversion element covers the sample application pad.
15. The device according to clause 14, wherein the liquid sample diversion area is surrounded by an enclosure, and an inner surface of the enclosure is provided with one or a plurality of grooves, so that the liquid from the liquid diversion element is capable of being discharged into, for example, the low-lying area at the bottom of the liquid diversion element by means of the grooves.
16. The device according to clause 15, wherein a length of the sample application pad is 14mm and a width thereof is 4mm, a length of the liquid diversion element is 17mm and a width thereof is 6mm, and therefore a width of the liquid diversion element covering the sample application area of each test strip is 6mm, and a distance between two test strips is 9mm.
17. The device according to clause 2, wherein a transition pad is further arranged downstream of the label pad, and the transition pad is located between the testing pad and the label pad.
18. The test device according to clause 4, wherein the first lateral flow testing element is used for testing COVID-19 antigens, and the second lateral flow testing element is used for testing influenza antigens.
19. The test device according to clause 2, wherein the upper card further comprises a window for exposing a testing area of the lateral flow testing element.
20. The test device according to clause 1, wherein the liquid sample is blood, saliva, nasal secretion, laryngeal secretion, or urine.

All the patents and publications mentioned in the description of the invention indicate that these are public technologies in the art and can be used by the invention. All the patents and publications cited herein are listed in the references, just as each publication is specifically referenced separately. The invention described herein can be realized in the absence of any one element or multiple elements, one restriction or multiple restrictions, where the limitation is not specifically described here. For example, the terms "comprising", "essentially consisting of" and "consisting of' in each example herein may be replaced by the rest 2 terms. The so-called "a/an" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the invention and appended claims. It can be understood that the examples described in the invention are some preferred examples and features. A person skilled in the art can make some modifications and changes according to the essence of the description of the invention. These modifications and changes are also considered to fall within the scope of the invention and the scope limited by independent claims and dependent claims.

## Claims

1. A device for testing an analyte in a fluid sample, comprising: a housing comprising an upper card and a lower card, wherein a supporting structure for supporting a lateral flow testing element is arranged on the lower card, the lateral flow testing element is configured to test the analyte in the fluid sample and comprises an application pad for receiving the liquid sample, the supporting structure comprises a supporting structure for supporting the entire sample application pad, and a part of the supporting structure for supporting the sample application pad is inclined.

2. The device according to claim 1, wherein the lateral flow testing element further comprises a label pad and a testing pad, wherein the label pad is located downstream of the application pad for receiving a liquid sample, and the other part of the supporting structure for supporting the sample application pad and a supporting structure for supporting the label pad are located in a substantially horizontal position.

3. The device according to claim 2, wherein a position of the inclined supporting structure is lower than that of the supporting structure for supporting the label pad.

4. The device according to claim 3, wherein the lateral flow testing element comprises a first lateral flow testing element having a first sample receiving pad and a second lateral flow testing element having a second sample receiving pad, wherein part of the inclined structure for supporting the sample application pad comprises a first inclined supporting structure and a second inclined supporting structure; and part of the first sample receiving pad covers the first inclined supporting structure, and part of the second sample receiving pad covers the second inclined supporting structure.

5. The device according to claim 4, wherein the first inclined supporting structure and the second inclined supporting structure are spaced apart in parallel.

6. The device according to claim 5, further comprising a liquid diversion element made of a water absorbent material, wherein the liquid diversion element covers the part of the first sample receiving pad and the part of the second sample receiving pad.

7. The device according to claim 6, wherein the lower card further comprises a liquid sample diversion area, the first inclined supporting structure and the second inclined supporting structure are located in the liquid sample diversion area, and the first inclined supporting structure and the second inclined supporting structure are distributed at two ends of the liquid sample diversion area.

8. The device according to claim 7, wherein the liquid diversion element is parallel to the first inclined supporting structure and the second inclined supporting structure or also obliquely arranged.

9. The device according to claim 7, wherein a liquid sample receiving area is arranged at the center of the liquid diversion element and located between the first inclined supporting structure and the second inclined supporting structure.

10. The device according to claim 6, wherein the liquid sample diversion area comprises a low-lying area, and liquid from the liquid diversion element obliquely arranged is capable of flowing to the low-lying area for collection.

11. The device according to claim 10, wherein the upper card comprises a liquid sample dripping hole, and the liquid sample dripping hole is located in the liquid sample receiving area of the liquid diversion element.

12. The device according to claim 11, wherein two sides of a sample application hole of the upper card are further provided with a diversion strip, respectively; and the diversion strip is in contact with the surface of the liquid diversion element.

13. The device according to claim 12, wherein the diversion strip is located on the first inclined supporting structure and in contact with the liquid diversion element on the second inclined supporting structure.

14. The device according to claim 13, wherein the upper card further comprises a pressing pin, and the pressing pin is arranged on the liquid diversion elements on the first inclined supporting structure and the second inclined supporting structure, respectively, so that the liquid diversion element covers the sample application pad.

15. The device according to claim 14, wherein the liquid sample diversion area is surrounded by an enclosure, and an inner surface of the enclosure is provided with one or a plurality of grooves, so that the liquid from the liquid diversion element is capable of being discharged into, for example, the low-lying area at the bottom of the liquid diversion element by means of the grooves.
